# EUROPEAN PATENT APPLICATION

(11) **EP 4 528 252 A1**
(43) Date of publication of application: **26.03.2025**
(21) Application number: 23918035.9
(22) Date of filing: 03.08.2023
(51) Int. Cl.: G01N 21/17, G01N 21/359, G01N 33/44

(54) **APPARATUS FOR DETERMINING TYPE OF OBJECT TO BE MEASURED**

(71) Applicant: Repla Inc., Gyeongsan-si, Gyeongsangbuk-do 38541 (KR)
(72) Inventor: SUH, Dong Eun, Suwon-si, Gyeonggi-do 16698 (KR); KIM, Min Sun, Changwon-si, Gyeongsangnam-do 51426 (KR); LEE, Hyun Jin, Seoul 04399 (KR); KIM, Rae Eun, Seongnam-si, Gyeonggi-do 13360 (KR)
(74) Representative: Vossius & Partner Patentanwälte Rechtsanwälte mbB
(86) International application number: PCT/KR2023/011377
(87) International publication number: WO 2025/028693

(57) **Abstract**

A device for determining the type of measurement object according to the present invention, which is a determining device that generates light toward a measurement object to recognize and determine the type of measurement object, includes a light detector formed by stacking a plurality of plates and configured to detect multiple beams of reflected light reflected from a measurement object and a sensor connected to the light detector and configured to determine the type of the measurement object through the reflected light detected by the light detector.

## Description

### [Technical Field]

The present invention relates to a device for determining the type of measurement object, and more particularly, to a device for determining the type of measurement object that can recognize as many measurement objects as possible using optical fibers and determine the type of the recognized measurement objects.

### [Background Art]

Representative examples of recyclable plastic include polyethylene terephthalate (PET), polyethylene (PE), polypropylene (PP), acrylonitrile butadiene styrene (ABS), and polyvinyl chloride (PVC).

Generally, an order of recycling plastic or plastic materials is simply as follows.

First, a waste company collects plastic and delivers the collected plastic to a collection company. Then, recyclable plastic is sorted through a sorting company. Then, the form of the sorted plastic is changed through an extrusion company and an injection company.

Here, plastic is a standardized raw material.

The conventional device for determining the type of measurement object is a device that recognizes measurement objects such as plastic flakes as individual materials, and in this state, for example, determines that, among thousand measurement objects, nine hundred are PET, fifty are polystyrene (PS), and fifty are PE.

However, since a large amount of impurities is caught in a filter mesh in the process of determining the type of plastic using the device for determining the type of measurement object, there is a problem in that accuracy in determining the type of plastic decreases, which causes a decrease in efficiency of recycling plastic. As a result, there is a problem in that the time and cost required for recycling plastic increase.

Also, in the case of the conventional device for determining the type of measurement object, it is difficult to sort a large amount of plastic all at once.

Consequently, when a large amount of plastic is sorted, there is a problem in that productivity decreases, and thus efficiency in determining the type of plastic and recycling plastic based on the determined type decreases.

Accordingly, there is a need for development of a device for determining the type of measurement object that can recognize as many plastic objects as possible and determine the type of the recognized plastic objects.

### [Disclosure]

### [Technical Problem]

The present invention is directed to providing a device for determining the type of measurement object that can determine the type of measurement object such as plastic with a minimum use of a sensor.

The present invention is also directed to providing a device for determining the type of measurement object that can determine a mixing ratio, which can be identified as more specific types of measurement objects such as plastic objects are determined, with a minimum use of a sensor.

The objectives of the present invention are not limited to the above-mentioned objectives, and other unmentioned objectives should be clearly understood by those of ordinary skill in the art from the description below.

### [Technical Solution]

The above objectives can be achieved by a device for determining the type of measurement object according to the present invention, which is a determining device that generates light toward a measurement object to recognize and determine the type of measurement object, the device including a light detector formed by stacking a plurality of plates and configured to detect multiple beams of reflected light reflected from a measurement object and a sensor connected to the light detector and configured to determine the type of the measurement object through the reflected light detected by the light detector.

Here, the light detector may be provided to detect the multiple beams of reflected light reflected from the measurement object and allow passage of some of the detected multiple beams of reflected light to transmit a detection result relating to the measurement object to the sensor.

The light detector may include a first plate to which a plurality of optical fibers are connected to detect the multiple beams of reflected light reflected from the measurement object and a second plate located above the first plate and configured to allow passage of a number of beams of reflected light fewer than a number of the multiple beams of reflected light detected by the first plate to transmit the detection result relating to the measurement object to the sensor.

Here, a through-hole configured to allow passage of the reflected light detected by the first plate may be formed to pass through the second plate.

The second plate may be provided to be rotatable, and the second plate may be provided to rotate in a predetermined direction to only allow passage of reflected light that coincides with the through-hole among the multiple beams of reflected light detected by the first plate.

Also, the second plate may be provided to rotate together with the sensor.

Meanwhile, the light detector may further include a rotating member connected to the second plate to rotate the second plate in the predetermined direction, and the second plate may be provided to only allow passage of the reflected light coinciding with the through-hole among the multiple beams of reflected light by rotating in the predetermined direction due to the rotating member.

Here, the rotating member may be provided as a step motor to control a rotational speed of the second plate.

Further, a light condensing member configured to gather the reflected light passing through the through-hole may be further provided at the through-hole.

Meanwhile, the light detector may further include an alignment housing configured to align the plurality of optical fibers connected to the first plate in a linear shape.

Here, the sensor may be provided to be connected to the second plate by at least one optical fiber.

Further, the light detector may further include a third plate disposed above the second plate and connected to the sensor to transmit reflected light that has passed through the second plate to the sensor.

Here, the third plate may be connected to the sensor by an optical fiber, and the optical fiber may include a first connector connected to the sensor and a second connector branched into a plurality of optical fibers from the first connector and connected to the third plate.

The light detector may be positioned inside a detector casing having a space therein, and one or more optical fibers may be provided to be exposed through at least any one of an upper end and a lower end of the detector casing.

The optical fiber exposed through the upper end of the detector casing may be provided to be connected to the sensor, and the optical fiber exposed through the lower end of the detector casing may be provided to detect the multiple beams of reflected light reflected from the measurement object.

Meanwhile, the sensor may include a near-infrared sensor.

### [Advantageous Effects]

A device for determining the type of measurement object according to the present invention has a light detector provided with a structure in which a plurality of plates are stacked, and thus can more effectively determine the type of measurement object such as plastic than the conventional devices for determining the type of measurement object. Further, the device for determining the type of measurement object according to the present invention has an advantage that it can efficiently determine a mixing ratio, which can be identified as more specific types of measurement objects such as plastic objects are determined, within a short time with a minimum use of a sensor.

Also, since the device for determining the type of measurement object according to the present invention has the light detector formed with the stacked structure, noise generated in the process of determining the type of measurement object can be reduced, thereby improving the reliability in determining the type of measurement object.

The advantageous effects of the present invention are not limited to those mentioned above, and other unmentioned advantageous effects should be clearly understood by those of ordinary skill in the art to which the present invention pertains from the description below.

### [Description of Drawings]

FIG. 1 is a perspective view of a device for determining the type of measurement object according to a first embodiment of the present invention.
FIG. 2 is a view illustrating a state in which the device for determining the type of measurement object illustrated in FIG. 1 is accommodated in a casing.
FIG. 3 is an exploded perspective view of the device for determining the type of measurement object illustrated in FIG. 1.
FIG. 4 is a perspective view of a device for determining the type of measurement object according to a second embodiment of the present invention.
FIG. 5 is an exploded perspective view of the device for determining the type of measurement object illustrated in FIG. 4.
FIG. 6 is a modification of the device for determining the type of measurement object illustrated in FIG. 4.
FIG. 7 is an exploded perspective view of the device for determining the type of measurement object illustrated in FIG. 6.

### [Modes of the Invention]

Hereinafter, embodiments of the present invention will be described in detail with reference to the accompanying drawings so that those of ordinary skill in the art to which the present invention pertains can easily carry out the embodiments. The present invention may be implemented in various different forms and is not limited to the embodiments described herein.

It is noted that the drawings are schematic, and are not necessarily drawn to scale. The relative dimensions and ratios of the components in the drawings may be exaggerated or diminished in size for the sake of clarity and convenience, and such arbitrary dimensions are merely illustrative and are not limitative. Furthermore, the same reference symbol is used for the same structure, element, or part shown in two or more drawings in order to represent similar features.

The embodiments of the present invention specifically illustrate ideal embodiments of the present invention. As a result, various modifications of the drawings are expected. Accordingly, the embodiments are not limited to the specific forms of illustrated regions, and include, for example, the modifications of shapes resulting from manufacture.

Hereinafter, devices 100 and 100-1 for determining the type of measurement object according to embodiments of the present invention (hereinafter referred to as "determining devices 100 and 100-1") will be described with reference to the accompanying drawings.

First, the determining devices 100 and 100-1 according to one embodiment of the present invention are for determining the types of measurement objects 102 such as plastic objects and determining a mixing ratio of the determined measurement objects 102.

Here, the types of the measurement objects 102 are determined by the determining devices 100 and 100-1 while the measurement objects 102 are placed on a table 101 or the like.

Also, although not illustrated in the drawings, the measurement objects 102 may be measured while located inside a box-shaped member having at least one closed surface, or the types of the measurement objects 102 may be determined by the determining device 100 while the measurement objects 102 are placed on a conveyor belt or the like moving in a predetermined direction.

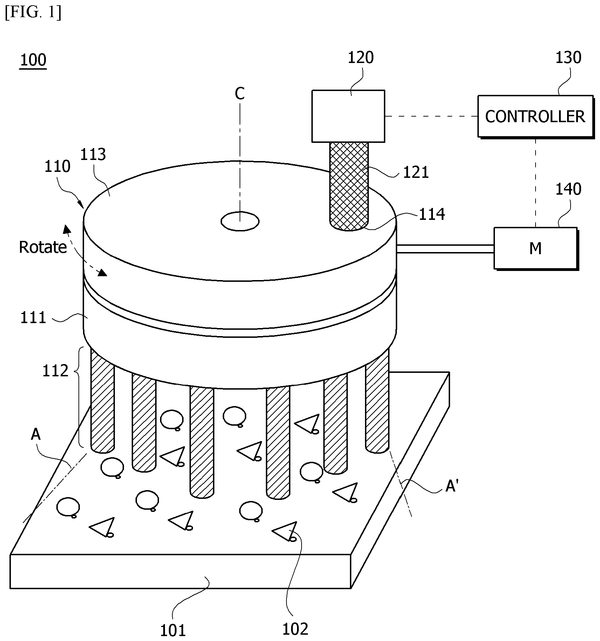

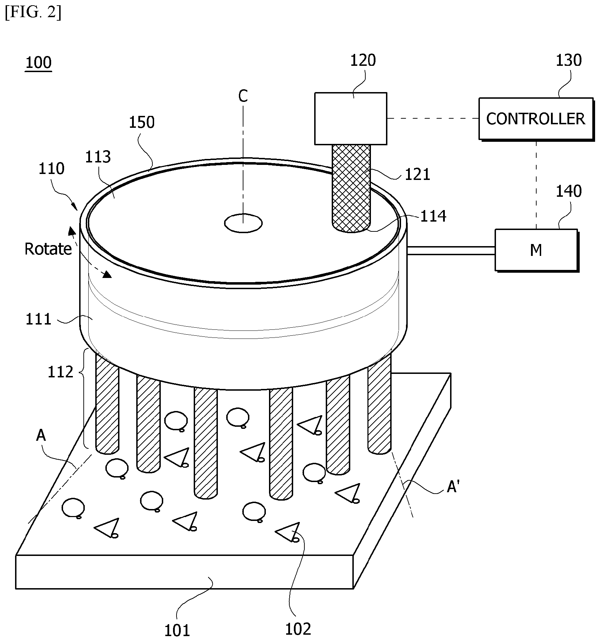

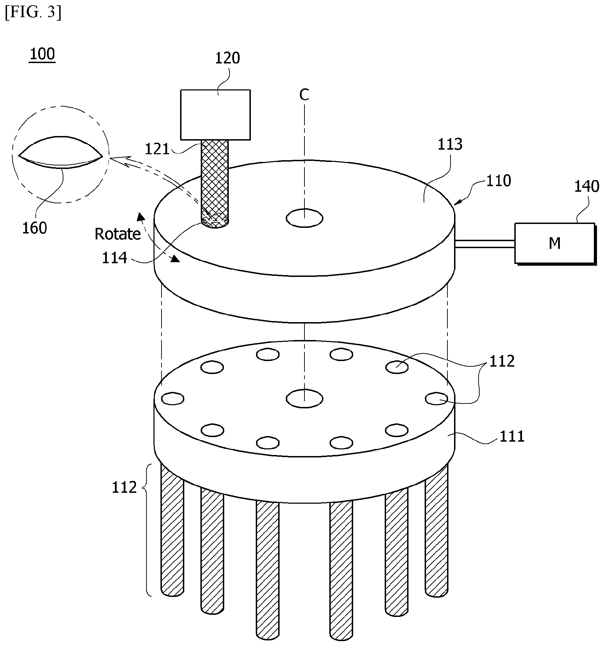

First, the determining device 100 according to a first embodiment of the present invention will be described with reference to FIGS. 1 to 3.

The determining device 100 according to the first embodiment of the present invention includes a light detector 110 and a sensor 120.

The light detector 110 is a part configured to detect multiple beams of reflected light reflected from the measurement objects 102.

Here, the measurement objects 102 are irradiated with light generated by a light generator (not illustrated) provided with halogen, a light emitting diode (LED), and the like. The light generated toward the measurement objects 102 by the light generator collides with one surface of the measurement objects 102 and then is reflected in a direction opposite to a direction in which the light is irradiated.

In this way, the light irradiated toward the measurement objects 102 by the light generator is reflected from multiple surfaces of each measurement object 102 as well as from the plurality of measurement objects 102 and causes multiple beams of light to be generated.

Here, the light detector 110 detects multiple beams of reflected light reflected from the measurement objects 102.

Here, the light detector 110 may detect all beams of reflected light reflected from the measurement objects 102 located in a detection region between A and A' as illustrated in the drawings.

The light detector 110 has a structure in which a plurality of plates 111 and 113 are stacked.

The sensor 120 may be connected to the light detector 110.

In particular, the sensor 120 may be connected to the uppermost side of the light detector 110.

Among the plurality of plates 111 and 113 included in the light detector 110, reflected light reflected from the measurement objects 102 is detected by the plate 111 located at the lowermost side, and the detected reflected light is transmitted to the plate 113 located at the uppermost side and the sensor 120 connected to the plate 113 at the uppermost side.

As described above, the sensor 120 may be used to determine the types of the measurement objects 102 by being connected to the light detector 110 and receiving the reflected light detected by the light detector 110.

For reference, in the determining device 100 according to the first embodiment of the present invention, the sensor 120 may be provided as a near-infrared (NIR) sensor but is not necessarily limited thereto.

Here, the sensor 120 may be connected to the light detector 110 using an optical fiber but is not necessarily limited thereto. Meanwhile, the determining device 100 according to the first embodiment of the present invention may further include a controller 130.

The controller 130 may be connected to the light detector 110 and the sensor 120.

Accordingly, the controller 130 receives a detection result of the measurement objects 102 that is detected by the light detector 110 and transmitted to the sensor 120. Also, the controller 130 may identify the types of the measurement objects 102, a mixing ratio thereof, and the like according to the received detection result of the measurement objects 102.

Further, the controller 130 may also control the operation of the light detector 110 formed by stacking the plurality of plates 111 and 113.

For example, at least one of the plurality of plates 111 and 113 of the light detector 110 may rotate, and the controller 130 may control a rotational force of the plate.

Meanwhile, in the determining device 100 according to the first embodiment of the present invention, the structure of the light detector 110 may vary according to the number of stacked plates 111 and 113.

Here, the light detector 110 detects multiple beams of reflected light reflected from the measurement objects 102 and allows passage of only some of the detected multiple beams of reflected light to transmit a detection result relating to the measurement objects 102 to the sensor 120.

Specifically, the light detector 110 according to the first embodiment of the present invention may include two plates 111 and 113.

The light detector 110 may include one first plate 111 and one second plate 113.

The first plate 111 is a part for detecting multiple beams of reflected light reflected from the measurement objects 102.

To this end, a plurality of optical fibers 112 may be connected to the first plate 111.

The plurality of optical fibers 112 may be connected to a lower end of the first plate 111.

The number of optical fibers 112 may vary, and preferably, about ten optical fibers 112 may be provided. Here, a measurement range of the measurement objects 102 placed on the table 101 may vary according to the number of provided optical fibers 112.

For reference, the range of measurement using ten optical fibers 112 may be the A-A' region including the entire region of the measurement objects 102 placed on the table 101 as illustrated in FIG. 1 but is not necessarily limited thereto.

The optical fibers 112 may be coupled to pass through the first plate 111 but may be coupled to be exposed only through the lower end of the first plate 111 without being exposed through the upper end thereof.

The second plate 113 is located above the first plate 111.

The second plate 113 allows passage of a number of beams of reflected light fewer than a number of the multiple beams of reflected light detected by the first plate 111, and a result of detecting the passed beams of reflected light is transmitted to the sensor 120.

To this end, at least one through-hole 114 may be formed in the second plate 113.

When any one of the multiple beams of reflected light detected by the first plate 111 coincides with the through-hole 114, the through-hole 114 allows passage of only the reflected light coinciding with the through-hole 114.

Meanwhile, a rotating member 140 may be connected to the second plate 113.

The rotating member 140 is a member configured to rotate the second plate 113 in a predetermined direction.

The rotating member 140 may be provided as a motor or the like but is not necessarily limited thereto and may be changed to any other member having a driving force that can rotate the second plate 113.

Also, the rotating member 140 may be provided as a step motor so that it is easy to control the rotational speed of the second plate 113.

For reference, although not illustrated in the drawings, the rotating member 140 may be directly connected to the second plate 113 and directly rotate the second plate 113. Also, the rotating member 140 may be indirectly connected to the second plate 113 via a movement transmission member (not illustrated) such as a belt and cause a rotational force to be generated at the second plate 113.

Accordingly, when the second plate 113 rotates in a predetermined direction due to the rotating member 140, only the reflected light coinciding with the through-hole 114 among the multiple beams of reflected light detected by the first plate 111 passes through the through-hole 114 and is transmitted to the sensor 120.

That is, in the light detector 110 according to the first embodiment of the present invention, the first plate 111 may be in a fixed state without rotating, and the second plate 113 may be in a rotatable state unlike the first plate 111.

Here, the first plate 111 and the second plate 113 are axially coupled, and in this state, a member such as a ball bearing (not illustrated) may be additionally formed on a coupling shaft and configured to be rotatable by receiving a driving force from the rotating member 140 to rotate only the second plate 113.

Also, when the second plate 113 rotates, the sensor 120 connected to the second plate 113 may also rotate together with the second plate 113.

Meanwhile, a light condensing member 160 may be additionally provided on the second plate 113.

The light condensing member 160 is for gathering multiple beams of reflected light into a single beam of reflected light to reduce loss of reflected light passing through the through-hole 114.

To this end, the light condensing member 160 may be located inside the through-hole 114 of the second plate 113 or may be located adjacent to any one of a lower end and an upper end of the through-hole 114.

Also, the light condensing member 160 may be provided as an optical fiber lens, a convex lens, or the like but is not necessarily limited thereto, and the type of light condensing member 160 may vary according to the shapes of the second plate 113 and the through-hole 114.

Meanwhile, although not illustrated in the drawings, the light detector 110 may further include an alignment housing (not illustrated).

The alignment housing is for aligning the plurality of optical fibers 112.

The alignment housing may be provided in the form of a hollow pipe but is not necessarily limited thereto.

Accordingly, the plurality of optical fibers 112 may be aligned in a linear shape by being inserted into the space inside the alignment housing.

Meanwhile, the light detector 110 according to the first embodiment of the present invention may be accommodated in a casing 150.

In other words, except for partial regions of the plurality of optical fibers 112 exposed through the lower end of the first plate 111, the first plate 111, the second plate 113, and the sensor 120 of the light detector 110 may be accommodated in the casing 150.

To this end, the casing 150 may include a hollow accommodation space (not illustrated) formed therein. Here, the accommodation space of the casing 150 may vary according to the shape and size of the light detector 110.

For reference, although the sensor 120 is illustrated in the drawings as not being accommodated in the casing 150, the present invention is not limited thereto, and the sensor 120 may either be accommodated or not accommodated in the casing 150 as necessary.

Meanwhile, in the case of the determining device 100 according to the first embodiment of the present invention, the plurality of optical fibers 112 may be exposed through a lower end of the casing 150.

The plurality of optical fibers 112 exposed through the lower end of the casing 150 detect multiple beams of reflected light reflected from the measurement objects 102.

For reference, an optical fiber (not illustrated) may be exposed through an upper end of the casing 150.

Here, the optical fiber exposed through the upper end of the casing 150 is a part connected to the sensor 120.

Also, the sensor 120 may be located inside the casing 150 instead of being exposed through the upper end of the casing 150 as described above. In this case, none of the components are located on the upper end of the casing 150.

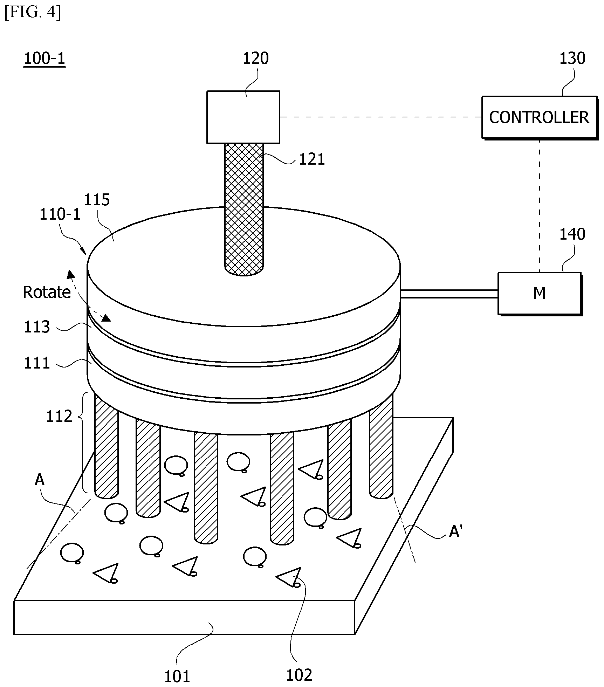

Hereinafter, the determining device 100-1 according to a second embodiment of the present invention will be described in detail with reference to FIGS. 4 to 7.

Since the determining device 100-1 according to the second embodiment of the present invention is substantially the same as the determining device 100 according to the first embodiment described above except for a structure of a light detector 110-1, the same names and reference numerals are assigned to the same components, and the above-described first embodiment may be referenced for descriptions thereof.

As illustrated in FIGS. 4 to 7, the determining device 100-1 according to the second embodiment of the present invention includes the light detector 110-1 and a sensor 120.

Here, the light detector 110-1 according to the second embodiment of the present invention may be provided with a structure in which a plurality of plates 111, 113, and 115 are stacked, and in particular, may include the three plates 111, 113, and 115.

The light detector 110-1 may be formed with a structure in which a first plate 111, a second plate 113, and a third plate 115 are sequentially stacked in this order.

The third plate 115 may be disposed above the second plate 113 and connected to the sensor 120. That is, the third plate 115 transmits reflected light that has passed through a through-hole 114 of the second plate 113 among multiple beams of reflected light detected by the first plate 111 to the sensor 120.

Here, the third plate 115 may be connected to the sensor 120 by an optical fiber 121.

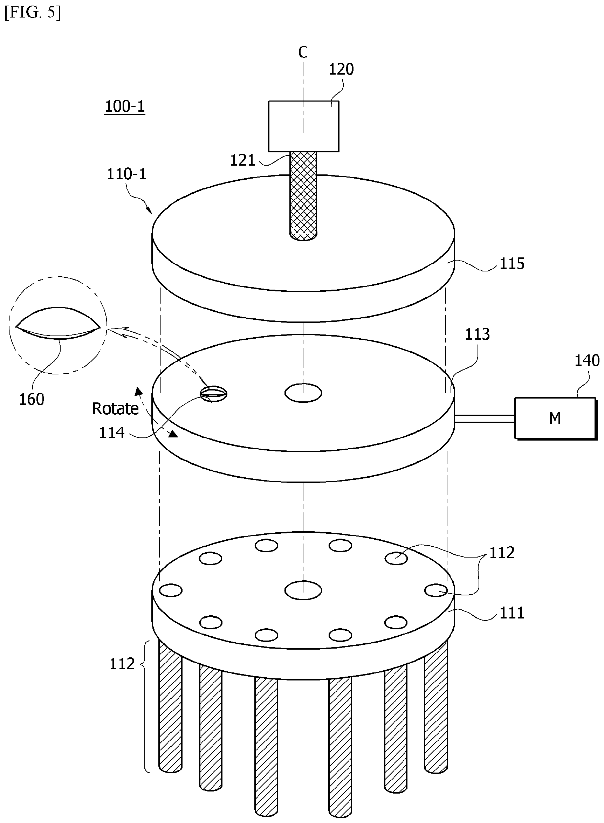

For example, as illustrated in FIGS. 4 and 5, the third plate 115 is connected to the sensor 120 by a single optical fiber 121.

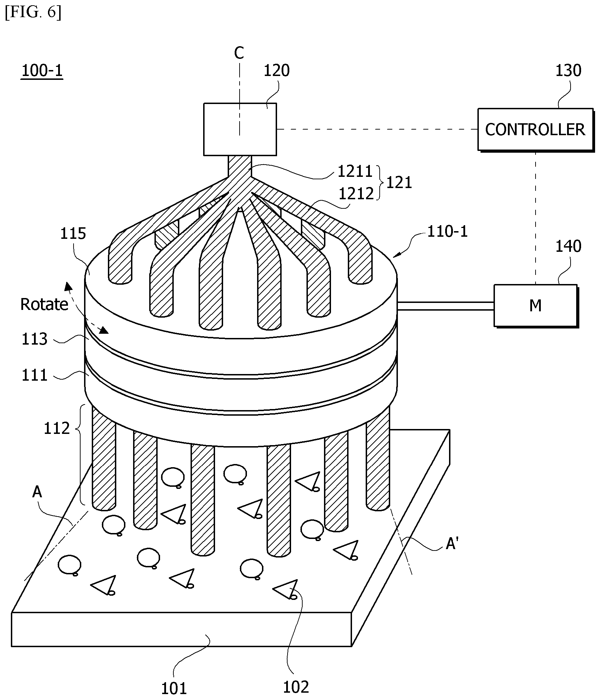

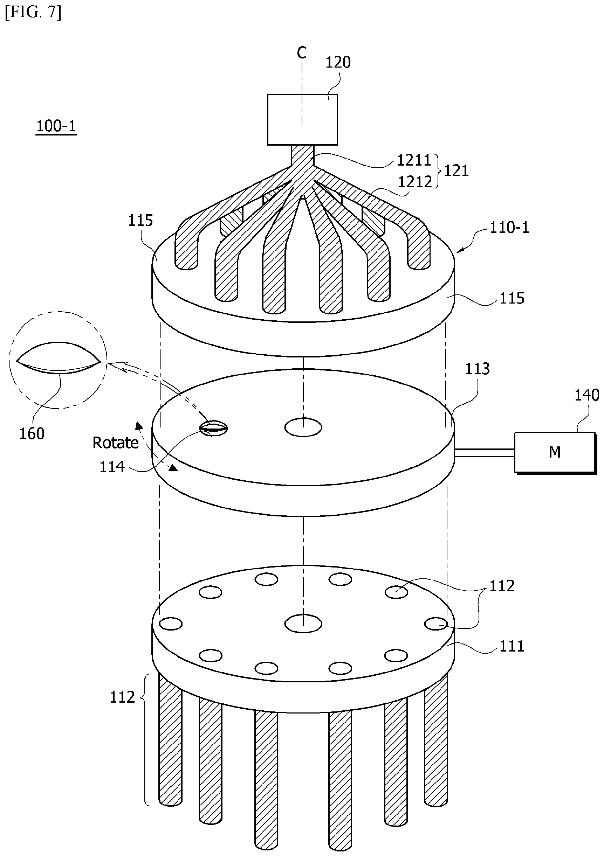

In another example, as illustrated in FIGS. 6 and 7, the third plate 115 may be connected to the sensor 120 by a single optical fiber 121 being branched into a plurality of optical fibers.

Here, the optical fiber 121 may include a first connector 1211 connected to the sensor 120 and a second connector 1212 branched into a plurality of optical fibers from the first connector 1211 and connected to the third plate 115.

Although the second connector 1212 is illustrated as being branched into ten optical fibers from the first connector 1211 in FIGS. 6 and 7, the second connector 1212 is not necessarily limited thereto, and the number of optical fibers into which the second connector 1212 is branched may vary.

Meanwhile, the second connector 1212 branched into ten optical fibers as described above may be connected to the third plate 115.

The second connector 1212 is provided to pass through an upper end of the third plate 115 but may be connected to not pass through a lower end thereof.

Meanwhile, although not illustrated in the drawings, in the case of the determining device 100-1 according to the second embodiment of the present invention, at least one or more optical fibers 112 and 121 may be exposed through each of an upper end and a lower end of a casing 150.

The optical fiber 121 exposed through the upper end of the casing 150 may be connected to the sensor 120. On the other hand, the optical fiber 112 exposed through the lower end of the casing 150 may be provided to detect multiple beams of reflected light reflected from measurement objects 102.

With the above configurations, the device 100 for determining the type of measurement object according to one embodiment of the present invention has the light detector 110 provided with the structure in which the plurality of plates 111 and 113 are stacked, and the device 100-1 for determining the type of measurement object according to another embodiment of the present invention has the light detector 110-1 provided with the structure in which the plurality of plates 111, 113, and 115 are stacked, and in this way, the type of measurement object 102 such as plastic can be more effectively determined than by the conventional devices for determining the type of measurement object. Further, there is an advantage in that a mixing ratio, which can be identified as more specific types of measurement objects 102 are determined, can be efficiently determined within a short time with a minimum use of the sensor 120.

Also, since the devices 100 and 100-1 for determining the type of measurement object according to the present invention have the light detectors 110 and 110-1 formed with the stacked structures, noise generated in the process of determining the type of measurement object 102 can be reduced, thereby improving the reliability in determining the type of measurement object 102.

The present invention has been described above using specific details such as specific components and some embodiments and drawings, but the above description is only provided to assist better overall understanding of the present invention, and the present invention is not limited to the above embodiments. Those of ordinary skill in the art to which the present invention pertains may make various modifications and changes to the above. Therefore, the spirit of the present invention should not be defined as being limited to the embodiments described above, and not only the claims below but also their equivalents and any modification derived from their equivalents should be construed as falling within the scope of the spirit of the present invention.

### [Industrial Applicability]

According to a device for determining the type of measurement object according to one embodiment of the present invention, the type of measurement object can be effectively determined using a light detector provided with a structure in which a plurality of plates are stacked.

## Claims

1. A device for determining the type of measurement object, which is a determining device that generates light toward a measurement object to recognize and determine the type of measurement object, the device comprising:
a light detector formed by stacking a plurality of plates and configured to detect multiple beams of reflected light reflected from a measurement object; and
a sensor connected to the light detector and configured to determine the type of the measurement object through the reflected light detected by the light detector.

2. The device of claim 1, wherein the light detector is provided to detect the multiple beams of reflected light reflected from the measurement object and allow passage of some of the detected multiple beams of reflected light to transmit a detection result relating to the measurement object to the sensor.

3. The device of claim 1, wherein the light detector includes:
a first plate to which a plurality of optical fibers are connected to detect the multiple beams of reflected light reflected from the measurement object;
a second plate located above the first plate and configured to allow passage of a number of beams of reflected light fewer than a number of the multiple beams of reflected light detected by the first plate; and
a third plate disposed above the second plate and connected to the sensor to transmit the reflected light that has passed through the second plate to the sensor,
wherein the third plate is connected to the sensor by an optical fiber, and
the optical fiber includes a first connector connected to the sensor and a second connector branched into a plurality of optical fibers from the first connector and connected to the third plate.

4. The device of claim 3, wherein a through-hole configured to allow passage of the reflected light detected by the first plate is formed to pass through the second plate.

5. The device of claim 4, wherein:
the second plate is provided to be rotatable; and
the second plate is provided to rotate in a predetermined direction to only allow passage of reflected light that coincides with the through-hole among the multiple beams of reflected light detected by the first plate.

6. The device of claim 5, wherein:
the light detector further includes a rotating member connected to the second plate to rotate the second plate in the predetermined direction; and
the second plate is provided to only allow passage of the reflected light coinciding with the through-hole among the multiple beams of reflected light by rotating in the predetermined direction due to the rotating member.

7. The device of claim 6, wherein the rotating member is provided as a step motor to be able to control a rotational speed of the second plate.

8. The device of claim 5, wherein a light condensing member configured to gather the reflected light passing through the through-hole is further provided at the through-hole.

9. The device of claim 3, wherein the light detector further includes an alignment housing configured to align the plurality of optical fibers connected to the first plate in a linear shape.

10. The device of claim 3, wherein:
the light detector is positioned inside a detector casing having a space therein; and
one or more optical fibers are exposed through at least any one of an upper end and a lower end of the detector casing.

11. The device of claim 10, wherein the optical fiber exposed through the upper end of the detector casing is provided to be connected to the sensor, and the optical fiber exposed through the lower end of the detector casing is provided to detect the multiple beams of reflected light reflected from the measurement object.

12. The device of claim 3, wherein the sensor includes a near-infrared sensor.
